(19) Europäisches Patentamt

European Patent Office

Office européen des brevets

(11) Publication number: **0 053 410**
**B1**

(12) **EUROPEAN PATENT SPECIFICATION**

(45) Date of publication of patent specification: **31.07.85**

(21) Application number: **81201233.4**

(22) Date of filing: **31.10.81**

(51) Int. Cl.⁴: **C 02 F 1/02, C 02 F 1/04,**
**C 07 C 126/00**

(54) **Process for the removal of urea, ammonia and carbon dioxide from dilute aqueous solutions.**

(30) Priority: **28.11.80 NL 8006477**

(43) Date of publication of application:
**09.06.82 Bulletin 82/23**

(45) Publication of the grant of the patent:
**31.07.85 Bulletin 85/31**

(84) Designated Contracting States:
**AT BE DE FR GB IT NL SE**

(56) References cited:
**GB-A-1 071 929**
**US-A-3 357 901**

**PATENTS ABSTRACTS OF JAPAN, vol. 2, no.**
**89, July 21, 1978 The Patent Office Japanese**
**Government. page 1468 C 78: Treating method**
**for aqueous solution containing urea,**
**ammonia and carbondioxide**
**CHEMICAL ENGINEERING PROGRESS, vol. 73,**
**no. 5, May 1977, AICHE New York, US D.F.**
**BRESS et al. "The startup of two major urea**
**plants", pages 80-84**

(73) Proprietor: **UNIE VAN KUNSTMESTFABRIEKEN**
**B.V.**
**Maliebaan 81**
**NL-3581 CG Utrecht (NL)**

(72) Inventor: **Zuidam, Jan**
**Beekerbaan 13 C**
**NL-6333 EH Schimmert (NL)**
Inventor: **Van Nassau, Petrus Johannes Marie**
**Burgemeester Pijlsstraat 4**
**NL-6151 HA Geleen (NL)**
Inventor: **Bruls, Pierre Gerard Marie Bernard**
**Schout Boutenstraat 21**
**NL-6121 HC Born (NL)**
Inventor: **Jonckers, Kees**
**Illikhoven 13**
**NL-6121 AH Born (NL)**

(74) Representative: **Roeffen, Wilhelmus Johannes**
**Maria et al**
**OCTROOIBUREAU DSM Postbus 9**
**NL-6160 MA Geleen (NL)**

## Description

The invention relates to a process for the removal of urea, ammonia and carbon dioxide from dilute aqueous solutions.

In the preparation of urea from ammonia and carbon dioxide a urea synthesis solution is formed at high temperature and at the pressure belonging to it, which solution still contains a substantial quantity of free ammonia and non-converted ammonium carbamate. The carbamate is decomposed, in one or more pressure steps, into ammonia and carbon dioxide, which are driven off, for the larger part, with the free ammonia present, and usually recirculated. In the final decomposition step an aqueous urea solution is obtained which still contains dissolved quantities of ammonia and carbon dioxide, which are subsequently removed by expansion to atmospheric or lower pressure. The aqueous urea solution is concentrated by evaporation and/or crystallization and further processed. During the evaporation a gas mixture is formed containing, beside water vapour, entrained fine urea drops and ammonia and carbon dioxide.

Like the gas mixture separated out in the expansion of the urea solution after the final decomposition step, this gas mixture is condensed, and the so-called process condensate thus obtained is partly returned into the process for absorbing the gas mixture carried off from the final decomposition step. The remaining part is discharged. The process condensate also incorporates the water fed into the process as steam for operating the ejectors in the evaporation section, washing water, flushing water on the stuffing boxes of the carbamate pumps, etc. Per mole of urea one mole of water is formed. So in a urea plant with a capacity of 1500 tonnes of urea per day 450 tonnes of water is formed per day. In addition about 300 tonnes of water per day is fed in, so that per day roughly 750 tonnes of water in all must be carried off.

The 2—9% by weight of $NH_3$, 0.8—6% by weight of $CO_2$ and 0.3—1.5% by weight of urea present in this water on the one side represent important quantities of raw materials and product and would, on the other side, load the surface water into which this waste water would be discharged to a degree no longer permitted by the governments in many countries. It is therefore necessary, before the discharge, to remove the larger part of the ammonia and the urea.

To this end the process condensate can be subjected to a treatment described in Industrial Wastes, September/October 1976 pages 44—47, in which treatment the process condensate already freed by desorption at low pressure of part of the ammonia and carbon dioxide is passed at higher pressure into the bottom of a reaction column and is heated herein by means of steam fed likewise to the bottom, owing to which urea will hydrolyze. The solution thus obtained is carried off from the top of the reaction column. In addition to a small quantity of non-hydrolyzed urea, it contains ammonia and carbon dioxide, which are removed, after expansion of the solution to the said low pressure, in a second desorption column by stripping with steam. The gas mixture thus separated off is used as stripping medium in the first desorption step. The bottom product of the second desorption column is discharged after heat exchange with the process condensate to be treated. Under practical circumstances this waste flow then still contains about 50 ppm $NH_3$ and 50 ppm urea. Also with very long residence times, for which inefficiently large reaction columns would be required, it is impossible to reach a urea content lower than 20—25 ppm.

Now, the object of the invention is to provide a process by means of which both the ammonia content and the urea content of dilute aqueous solutions can be reduced to lower than 10 ppm. To this end a combination of features is applied comprising:

1. The urea-containing solution which is subjected to the hydrolysis treatment contains amounts of ammonia and carbon dioxide which have been reduced as far as possible.

2. The urea-containing solution is countercurrently contacted with an inert gas that provides the heat required for the hydrolysis and simultaneously acts as stripping agent, so that the ammonia and carbon dioxide resulting from the hydrolysis reaction are expelled from the solution.

3. The gaseous mixture containing ammonia and carbon dioxide thus obtained in the hydrolysis is discharged as soon as it is formed. So, the invention relates to a process for the removal of urea, ammonia and carbon dioxide from dilute aqueous solutions comprising removal of ammonia and carbon dioxide in a pre-desorption zone at a pressure of 1—5 bar, hydrolysis of urea in a reaction column at a pressure of at least 10 bar while supplying heat followed by desorption of ammonia and carbon dioxide from the aqueous solution discharged from the reaction column. The process is characterized in that the solution obtained in the pre-desorption zone is passed at a pressure of up to 30 bar into the top of a reaction column and is caused to flow downward therein countercurrently to a gas stream the heat content of which is sufficient to maintain in the top of the reaction column a temperature of between 170 and 220°C and in the bottom of the reaction column a temperature of between 180 and 230°C, a gas mixture containing ammonia, carbon dioxide and water vapour is carried off from the top of the reaction column and a substantially urea-free aqueous solution of ammonia and carbon dioxide is discharged from the bottom of the reaction column, and subsequently, at a pressure of 1—5 bar, ammonia and carbon dioxide are removed from the solution discharged from the reaction column. The ammonia and carbon dioxide must be removed from the solution to be treated to such a degree that in the bottom zones of the reaction column, where the urea concentrations are low, further hydrolysis of urea will not be impeded. The gas mixture containing the ammonia and carbon dioxide formed in the hydrolysis treatment, is removed from the liquid phase in the reaction column as soon as it is formed. This gas mixture can be used as heating and stripping agent in the pre-desorption zone.

The heating and stripping agent to be applied in the reaction column is preferably steam of 15—42 bar. It is true that other gases can be used as well, but they must be separated off again, which will involve extra costs.

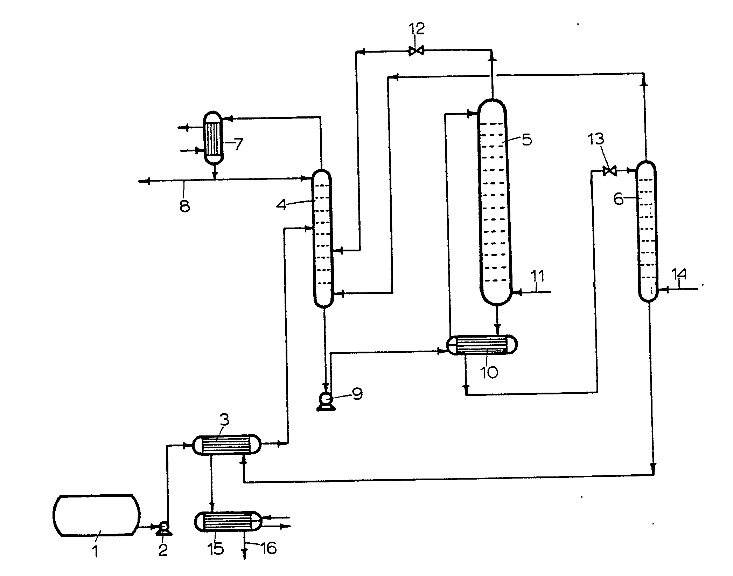

The invention will subsequently be elucidated by means of the attached drawing.

In it the treatment of the process condensate formed in the preparation of urea is represented diagrammatically.

The process condensate collected in tank 1 is brought to a pressure of 1—5 bar, for instance 3—4 bar, by means of pump 2 and passed, via heat exchanger 3, into the bottom of the top half of pre-desorption column 4. Herein the larger part of the dissolved ammonia and carbon dioxide is driven out by the stripping effect of gas flows passed into the bottom half from reaction column 5 and desorption column 6 and the heat present in these gas flows. The gas mixture thus separated off, which also contains a quantity of water vapour, is condensed completely in reflux condenser 7. A small part of the condensate is returned to the top of pre-desorption column 4, the larger part is passed, through line 8, to the urea synthesis, for instance to the condensation and absorption zones of the final decomposition step. If desired the whole amount of the condensate can be passed to the urea synthesis.

The solution treated in pre-desorption column 4, which solution still contains, in addition to small quantities of ammonia and carbon dioxide, virtually the whole quantity of urea originally present, subsequently passed by means of pump 9 under a pressure of 10—30 bar, for instance 12.5 bar, via heat exchanger 10 into the top of reaction column 5. Reaction column 5 is divided, for instance by sieve plates, into a number of compartments which serve as ideal bubble gas washers. Into the bottom of this reaction column a gas stream, in the embodiment shown here steam of 15—30 bar, for instance 25 bar, is passed via 11. The temperature variation over the length of the column is set by control of the quantity of steam and the pressure in the reaction column so that the top temperature is between 170 and 220°C and the bottom temperature between 180 and 230°C. At the said pressure of 12.5 bar a top temperature of 181°C and a bottom temperature of 193°C are applied. The average temperature will then be about 185°C. The steam supplies the heat necessary for the hydrolysis of urea and the evaporation of the ammonia and carbon dioxide released in this process. The urea content of the solution flowing down in reaction column 5 decreases fairly rapidly and that more rapidly as the temperature is higher. It has been found that after a certain residence time the final urea content is determined by the ammonia and carbon dioxide concentrations in the liquid. For this reason the pre-desorption of ammonia and carbon dioxide is often necessary to reach the required low urea content of 10 ppm or lower. At an average temperature of 185°C a minimum residence time in the reaction column of 70 minutes is necessary for instance. As the average temperature in the reaction column is higher, a shorter residence time will suffice.

The mixture of gases driven off and stripping agent formed in reaction column 5 is carried out from the top and after expansion in reducing device 12 to the pressure at which pre-desorption column 4 is operated, in the embodiment of the process according to the invention described here 3—4 bar, passed into pre-desorption column 4 at some distance below the supply of the process condensate, in which column it gives off part of its heat content to the liquid flowing down.

The liquid which collects in the bottom of reaction column 5, and which is freed almost entirely of urea, gives off heat, in heat exchanger 10, to the feed of this column, is subsequently expanded in reducing device 13 to a pressure of 1—5 bar, for instance 3.5 bar, and passed into the top of desorption column 6. The mixture of gaseous ammonia, carbon dioxide and water vapour released in the expansion is immediately separated off herein, and the remaining liquid phase flows down, in the desorption column, countercurrently to a quantity of steam supplied at 14, whose heat content is sufficient for the evaporation of ammonia and carbon dioxide and whose stripping capacity is, moreover, sufficient to reach the required ammonia content of 10 ppm or lower. The ammonia and carbon dioxide thus driven off and the entrained water vapour are passed, with the gas mixture separated off in the top of the desorption column, into the bottom of pre-desorption column 4 and serve, in this column, as heating and stripping agents for the liquid to be treated.

From the bottom of desorption column 6 a flow of waste is carried out with urea and ammonia concentrations of 10 ppm or lower. Part of the heat present in this flow is used in heat exchanger 3 for pre-heating the liquid to be treated in pre-desorption column 4. The waste water flow is then optionally cooled in cooler 15 with cooling water and finally discharged through line 16.

### Example

The process condensate obtained in a urea plant with a production of 1500 tonnes a day was treated by means of the process described above. The quantities are given in kg per hour.

The process condensate, 28,333 kg, contained 4.38% by weight of $NH_3$, 2.95% by weight of $CO_2$ and 1.09% by weight of urea and was heated in heat exchanger 3 from 42°C to 125°C. In pre-desorption column 4 this solution was first brought countercurrently, at a pressure of 3.43 bar, into contact with 1147 kg gas mixture from reaction column 5, which consisted of 66 kg $NH_3$,

252 kg $CO_2$ and 829 kg water vapour and which has a temperature of 181°C, and subsequently with 5562 kg gas mixture from desorption column 6, the composition of which was: 421 kg $NH_3$, 5 kg $CO_2$ and 5136 kg water vapour and the temperature of which was 136°C. Via the top of column 4 2293 kg $NH_3$, 1720 kg $CO_2$ and 2528 kg water vapour were carried off. This gas mixture was condensed completely and of the solution thus obtained, which has a temperature of 54°C, part, consisting of 876 kg $NH_3$, 657 kg $CO_2$ and 966 kg $H_2O$, was returned as reflux into the desorption column. The remaining part, which contained, beside 1562 kg $H_2O$, 1417 kg $NH_3$ and 1063 kg $CO_2$, was returned to the urea plant.

So in the pre-desorption step already 75% of the original quantity of $NH_3$ had been removed.

Beside 30,349 kg water, the desorbed solution contained 310 kg $NH_3$, 31 kg $CO_2$ and 319 kg urea, was brought by means of pump 9 to a pressure of 12.26 bar and passed, after heating from 135°C to 183°C in heat exchanger 10, into the top of reaction column 5. The quantity of steam passed into the bottom of this reaction column was 1700 kg, its temperature was 225°C and the pressure 24.5 bar. The urea present in the feed was hydrolyzed almost completely to $NH_3$ and $CO_2$. A gas mixture with the said composition was carried out and expanded from 14.7 bar to 3.43 bar.

The bottom product from the reaction column consisted of 31,127 kg water, 419 kg $NH_3$ and 6 kg $CO_2$ and was used for pre-heating the feed stream of the reaction column, in which process the temperature fell from 193°C to 146°C. By expansion in the top of description column 6, the pressure was lowered again to 3.43 bar, in which process the temperature further decreased to 136°C. In this desorption column this solution was stripped with 5000 kg steam of 147°C. In this process the said 5562 kg gas mixture was formed, which was passed into the bottom of the pre-desorption column. The water carried off from the bottom of desorption column 6 was used first for pre-heating the process condensate to be treated, in which treatment the temperature fell from 139°C to 63°C, and was subsequently cooled with cooling water to 40°C, upon which it was carried off. It contained 6 ppm $NH_3$ and 8 ppm urea.

## Claims

1. Process for the removal of urea, ammonia and carbon dioxide from dilute aqueous solutions comprising removal of ammonia and carbon dioxide in a pre-desorption zone at a pressure of 1—5 bar, hydrolysis of urea in a reaction column at a pressure of at least 10 bar while supplying heat followed by desorption of ammonia and carbon dioxide from the aqueous solution discharged from the reaction column, characterized in that the solution obtained in the pre-desorption zone is passed at a pressure of up to 30 bar into the top of the reaction column and caused to flow downward therein countercurrently to an inert gas stream the heat content of which is sufficient to maintain in the top of the reaction column a temperature of between 170 and 220°C and in the bottom of the reaction column a temperature of between 180 and 230°C, a gas mixture containing ammonia, carbon dioxide and water vapour is carried off from the top of the reaction column and a substantially urea-free aqueous solution of ammonia and carbon dioxide is discharged from the bottom of the reaction column, and subsequently, at a pressure of 1—5 bar, ammonia and carbon dioxide are removed from the solution discharged from the reaction column.

2. Process according to claim 1, characterized in that in the reaction column steam is used as the said gas stream.

3. Process according to claim 2, characterized in that steam with a pressure of 15—42 bar is used.

4. Process according to any one of the claims 1—3, characterized in that ammonia and carbon dioxide are removed from the solution carried off from the bottom of the reaction column by countercurrently stripping with steam.

5. Process according to any one of the claims 1—4, characterized in that the solution to be fed to the reaction column is pre-heated by heat exchange with the solution carried off from the bottom of the reaction column.

6. Process according to any one of the claims 1—5, characterized in that, after expansion to a pressure of 1—5 bar, the gas mixture carried off from the top of the reaction column is passed into the pre-desorption zone.

7. Process according to any one of the claims 1—6, characterized in that the gas mixture containing the ammonia and carbon dioxide separated from the solution carried off from the bottom of the reaction column is passed into the pre-desorption zone.

8. Process according to any one of the claims 1—7, characterized in that the gas mixture carried off from the pre-desorption is condensed and the dilute aqueous solution of ammonia and carbon dioxide thus obtained is worked-up, at least in part, in the preparation of urea.

**Patentansprüche**

1. Verfahren zum Entfernen von Harnstoff, Ammoniak und Kohlendioxid aus verdünnten wässerigen Lösungen, bestehend aus dem Entfernen von Ammoniak und Kohlendioxid in einer Vordesorptionszone bei einem Druck von 1—5 bar, Hydrolyse von Harnstoff in einer Reaktionssäule bei einem Druck von mindestens 10 bar, unter Zuführung von Wärme mit nachfolgender Desorption von Ammoniak und Kohlendioxid aus der wässerigen Lösung, die aus der Reaktionskolonne abgeführt wurde, dadurch gekennzeichnet, dass die in der Vordesorptionszone erhaltene Lösung bei einem Druck bis zu 30 bar oben in die Reaktionskolonne eingeführt wird und hierin im Gegenstrom zu einem Inertgasstrom nach unten fliesst, dessen Wärme ausreicht, im Kopf der Reaktionskolonne eine Temperatur zwischen 170 und 220°C und am Boden der Reaktionskolonne eine Temperatur von 180 bis 230°C aufrechtzuerhalten, vom Kopf der Reaktionskolonne ein Ammoniak, Kohlendioxid und Wasserdampf enthaltendes Gasgemisch und vom Boden der Reaktionskolonne eine im wesentlichen harstofffreie wässerige Lösung von Ammoniak und Kohlendioxid abgeführt werden und anschliessend bei einem Druck von 1—5 bar aus der aus der Reaktionskolonne abgeführten Lösung Ammoniak und Kohlendioxid entfernt werden.

2. Verfahren nach Anspruch 1, dadurch gekennzeichnet, dass der Gasstrom in der Reaktionskolonne Dampf ist.

3. Verfahren nach Anspruch 2, dadurch gekennzeichnet, dass Dampf mit einem Druck von 15—42 bar verwendet wird.

4. Verfahren nach Anspruch 1 bis 3, dadurch gekennzeichnet, dass aus der aus dem Boden der Reaktionskolonne abgeführten Lösung Ammoniak und Kohlendioxid durch Strippen mit Dampf im Gegenstrom entfernt werden.

5. Verfahren nach Anspruch 1 bis 4, dadurch gekennzeichnet, dass die der Reaktionskolonne zuzuführende Lösung durch Wärmeaustausch mit der aus dem Boden der Kolonne abgeführten Lösung vorerwärmt wird.

6. Verfahren nach Anspruch 1 bis 5, dadurch gekennzeichnet, dass das aus dem Kopf der Reaktionskolonne abgeführte Gasgemisch nach Expansion auf einen Druck von 1—5 bar in die Vordesorptionszone eingeleitet wird.

7. Verfahren nach Anspruch 1 bis 6, dadurch gekennzeichnet, dass das aus der Bodenlösung der Reaktionskolonne abgeschiedene ammoniak- und kohlendioxidhaltige Gasgemisch in die Vordesorptionszone eingeführt wird.

8. Verfahren nach Anspruch 1 bis 7, dadurch gekennzeichnet, dass das aus der Vordesorption abgeführte Gasgemisch kondensiert und die so anfallende verdünnte wässerige Lösung aus Ammoniak und Kohlendioxid wenigstens teilweise bei der Harnstofferzeugung aufgearbeitet wird.

**Revendications**

1. Procédé d'élimination d'urée, d'ammoniac et de dioxyde de carbone de solutions aqueuses diluées comportant l'élimination d'ammoniac et de dioxyde de carbone dans une zone de prédésorption à une pression de 1 à 5 bars, l'hydrolyse d'urée dans une colonne de réaction à une pression d'au moins 10 bars, tout en apportant la chaleur et la désorption de l'ammoniac et du dioxyde de carbone de la solution aqueus évacuée de la colone de réaction, caractérisé en ce que la solution obtenue dans la zone de prédésorption est introduite à une pression jusqu'a 30 bars en haut de la colonne de réaction et est amenée à s'y écouler vers le bas à contre-courant d'un courant gazeux, la chaleur de se courant gazeux étant suffisante pour maintenir en haut de la colonne de réaction une température comprise entre 170 et 220°C et en bas de la colonne de réaction une température comprise entre 180 et 230°C, alors que du sommet de la colonne, on évacue un mélange de gaz contenant de l'ammoniac, du dioxyde de carbone et de la vapeur d'eau et que du fond de la colonne, on évacue une solution aqueuse d'ammoniac et de dioxyde de carbone, pratiquement exempte d'urée, et qu'ensuite on élimine de cette solution, évacuée du fond de la colonne, l'ammoniac et le dioxyde de carbone à une pression de 1 à 5 bars.

2. Procédé selon la revendication 1, caractérisé en ce que le courant de gaz dans la colonne de réaction est de la vapeur.

3. Procédé selon la revendication 2, caractérisé en ce qu'on utilise de la vapeur ayant une pression de 15—42 bars.

4. Procédé selon l'une des revendications 1 à 3, caractérisé en ce qu'on élimine de la solution évacuée du fond de la colonne de réaction l'ammoniaque et le dioxyde de carbone par entraînement à la vapeur à contre-courant.

5. Procédé selon l'une des revendications 1 à 4, caractérisé en ce que la solution à amener à la colonne de réaction est préchauffée par échange de chaleur avec la solution évacuée du fond de la colonne de réaction.

6. Procédé selon l'une des revendications 1 à 5, caractérisé en ce que le mélange de gas évacué du sommet de la colonne de réaction est détendu jusqu'à une pression de 1—5 bars et puis introduit dans la zone de prédésorption.

7. Procédé selon l'une des revendications 1 à 6, caractérisé en ce que le mélange de gaz contenant de l'ammoniaque et du dioxyde de carbone, qui est séparé de la solution de fond de la colonne de réaction, est introduit dans la zone de prédésorption.

8. Procédé selon l'une des revendications 1 à 7, caractérisé en ce que le mélange de gaz évacué de la prédésorption est condensé et que la solution aqueuse diluée d'ammoniaque et de dioxyde de carbone ainsi obtenue est traitée au moins partiellement lors de la préparation d'urée.